# EUROPEAN PATENT SPECIFICATION

(11) **EP 2 779 955 B1**
(45) Date of publication and mention of the grant of the patent: **29.06.2016**
(21) Application number: 12805827.8
(22) Date of filing: 19.11.2012
(51) Int. Cl.: A61F 2/78, A61F 2/50

(54) **PROSTHETIC LINER**
PROTHESENEINLAGE
MANCHON POUR PROTHÈSE

(30) Priority: 18.11.2011 US 201113299532
(43) Date of publication of application: 24.09.2014
(73) Proprietor: Otto Bock HealthCare GmbH, 37115 Duderstadt (DE); Pianykh, Oleg, Salt Lake City, UT 84121 (US); Polta, Charles, C., Salt Lake City, UT 84121 (US); Bussiek-Cillien, Kai, 37075 Gottingen (DE); Schneider, Scott, John, Sartell, MN 56377 (US)
(72) Inventor: PIANYKH, Oleg, Salt Lake City, UT 84121 (US); POLTA, Charles, C., Salt Lake City, UT 84121 (US); BUSSIEK-CILLIEN, Kai, 37075 Gottingen (DE); SCHNEIDER, Scott, John, Sartell, MN 56377 (US)
(74) Representative: Lins, Edgar
(86) International application number: PCT/US2012/065888
(87) International publication number: WO 2013/075115

(56) References cited:
- SU-A1- 1 739 990
- US-A- 6 149 691
- US-A1- 2005 240 283
- US-A1- 2007 061 017
- US-A1- 2011 054 635
- US-A1- 2011 118 854
- US-B1- 6 362 387
- US-B1- 6 440 345

## Description

### Technical field

The invention relates to a below knee amputee prosthetic liner according to the preamble of claim 1.

Such a liner is known from US 6,362,387 B1.

In addition, the present invention relates to a method for producing a liner of this sort.

### Background of the invention

The use of prosthetic liners for amputees is well known from the prior art. A prosthetic liner is worn between the amputation limb and the prosthesis socket, carrying the lower leg portion of the prosthesis. The liner is worn directly on the amputation limb. At the closed distal end of the liner body the liner can be provided with means for connecting the liner to the prosthesis socket. These means might for example be threaded inserts, which can be moulded into the polymer like liner material, in particular by injection moulding.

Alternatively the liner can be used in a prosthetic system in which at least a part
of a volume between the liner and a prosthetic socket is isolated from the surrounding atmosphere. During standing air is removed from this volume through an exhaust valve. In a swing phase a partial vacuum or negative pressure occurs in the volume, holding the prosthesis at the amputation limb. As an alternative to the exhaust valve a vacuum can be generated in the volume between the liner and the prosthetic socket due to which the amputation limb is sucked into the prosthetic socket. The isolation of the volume is usually achieved by means of a seal at the prosthetic socket or at the liner or by means of a so called sleeve, which is unrolled over socket and liner, thereby sealing the distal end.

Since the liner usually is the only part of the prosthesis getting in direct contact with the skin of the amputee and the liner is usually worn the whole day, it is important to make the liner as comfortable to wear as possible. The liners might be for example constructed either of a fabric impregnated with silicone or another polymer like material or from the polymer like material only.

From US 6,440,345 B1 and US 6,918,936 B2 a liner is known, which comprises a knee part having a pre-flexed angle. This is also known from US 5,888,216. Compared with the straight tubular shape liner, this pre-flexed angle minimizes the wrinkling behind the knee, especially when the knee is bent. In addition it increases the range of motion of the knee joint and is more comfortable to wear when sitting.

Since the diameter of the amputation limb of the person usually decreases towards the distal end of the amputation limb, the distal part of the liner according to US 5,888,216 is tapered. This leads to a better fit and a more homogeneous distribution of pressure at the amputation limb of the person.

From US 2010/0016993 A1 a liner is known, which also shows a knee part, in which a strip or a tendon is attached to urge the elastomeric material in this region of the tubular liner body to contract. This strip or tendon is for example of silicone having a higher durometer hardness value than the material of the liner body and may be for example integrally moulded with the knee portion of the tubular liner body.

In order to provide a perfect fit of the liner at the residual limb of the person it is possible to use a fully individualized liner or to individualize a standard liner, as for example is known from WO 2009/109182. Since the construction of an individual liner is time-consuming and expensive it is much cheaper and faster to provide some standard liners which may be available in different sizes and to use these standard liners for each amputation limb.

Unfortunately, amputation limbs are shaped very individually. When a standard liner is used it is possible that cavities occur between the amputation limb of the person and the liner. This can lead to swelling or chafing at the residual limb. In order to prevent at least most of these cavities from occurring between the liner and the residual limb it is known for example from US 2005/0240283 A1 to insert some cushions into the liner. This leads to a more homogeneous distribution of pressure at the surface of the amputation limb covered by the liner.

It is known from several documents to insert these cushions into the liner. While US 2011/0118854 A1 teaches to use thickenings on the outside of the liner body which does not get in contact with the skin of the user, it is known from several other documents to use thickenings between an inner liner and an outer liner. This is known from US 6,149,651, US 6,362,387 B1, SU 173 99 90 A1 and US 2007/0061017 A1. US 2011 /54635 A1 teaches to use one large thickening of the liner body in the region of the proximal end in order to cushion this end when the liner is positioned inside a prosthetic shaft.

At the distal end of the liner there is usually a means for connecting the liner to the remaining parts of the prosthesis. Silicone liners particularly utilize a hard unyielding material such as aluminum or thermoplastics such as polycarbonate or polyamids as a shuttle screw housing for fixing the lower leg portion of the prosthesis. The said means can also be a screw housing or a threaded insert which might be moulded into the material of the liner body. In order to prevent these hard materials from being felt by the wearer of the prosthesis liner, the thickness of the moulded polymer like liner material is considerably enhanced at the closed distal end of the liner body. In addition, the amputation limb is very sensitive in this region due to the strong pressure during use of the prosthesis weighing on the end of the respective bone in the distal end of the liner.

In order to increase the wearing comfort of the prosthesis even further, it is known to insert some cushions into the prosthetic socket, which is to be fixed to the liner. Since the prosthetic socket is made from a rigid material, this can lead to bruising and chafing and hence to discomfort and pain.

These cushions are usually positioned at an inner surface of the prosthetic socket. This leads to dirt pockets making the cleaning of the prosthetic socket difficult and time-consuming. In addition it is difficult to position these cushions at the inner surface of the prosthesis socket exactly. This might lead to wrong positions of the cushions so that the desired cushioning effect is not realized or at least is not realized at the desired locations at the amputation limb. This again leads to chafing, pressure points, swelling and pain.

### Objects of the invention

An object of the invention is thus to provide a prosthetic liner which increases the wearing comfort of the prosthesis and simultaneously ensures that the prosthetic liner as well as a prosthetic socket that should be attached to the liner are easy to clean. Another object of the invention is to provide a method for producing such a liner.

### Summary of the invention

In accordance with the present invention, there is provided a prosthetic liner as defined in independent claim 1. A method for producing such a liner is defined in independent claim 6. Preferred embodiments are defined in the dependent claims.

Due to the fact that the thickenings are parts of the prosthetic liner and not positioned on the prosthetic socket in the form of cushions, an exact positioning relative to an amputation limb received in the liner body is easily and comfortably achieved. By using different materials, the various requirements can be met. In this way, it is conceivable that the thickening can be made from a softer material than the liner body. In this case, a cushioning effect caused by the thickenings is further reinforced. At the same time, the stability and the quality of the fit of the amputation limb in the prosthetic liner are not affected, as the liner body can be made from a material that is optimal for this purpose.
In one advantageous embodiment, the at least one thickening is formed by at least one pad which is arranged on the liner body. In this way, an especially simple positioning of the pad is possible. In addition, the liner body can be used in one of a number of standard shapes or moulds that are, for example, available in various sizes and later can be individualized to the wearer and shape of the amputation limb by attaching the corresponding pad.

In accordance with the present invention the prosthetic liner is a below knee amputee prosthetic liner wherein the first region is adapted to cover a head of the fibula of the amputation limb and the second region is adapted to cover a shinbone of the amputation limb, once the amputation limb is received in the liner body.
The at least one thickening provides a cushioning effect for the head of the fibula and/or the shinbone of the amputation limb. Because of this additional cushioning of the prominent bones of the amputation limb the liner is very comfortable to wear. In addition, there are no more cushions to be provided at an inner surface of a prosthetic socket, which is to be attached to the prosthesis liner. Hence, the inner surface of a prosthetic socket can be flat and smooth making it is easier to clean. This of course also applies to all other prosthetic liners according to the invention.

According to the present invention the liner is symmetric with respect to a plane of symmetry in which the knee of the amputation limb is bendable, once the amputation limb is received in the liner body. The plane of symmetry is the so called sagittal plane. By this it is possible to use the same liner for a below knee amputation limb of a right leg and of a left leg. The liner hence has two thickenings for covering the head of the fibula, one of which is provided on the right hand side of the liner body and the other one is provided is one the left hand side, i.e. on the medial and lateral sides.

In another embodiment not covered by the present invention the liner is a thigh liner. In this case a thickening can be arranged at the lateral side in the distal region of the liner. This thickening can extend from the distal end of the liner towards the open proximal end thereof and can have more than half of the length of the liner. The thickening can be arranged on the lateral side only. Alternatively it can be arranged in a rotationally symmetric manner around the full circumference of the liner. In yet another embodiment two thickenings can be arranged at the lateral side and the medial side of the liner, respectively. In this case the liner is symmetric with respect to the sagittal plane and hence can be used for both a right and a left leg amputee.

The thickening in the lateral femur area leads to a cushioning which is particularly advantageous for atrophied amputation limbs which have a low soft tissue covering. In an abduction position of the femur the thickening provides an additional positioning of the femur by shifting or pushing it in medial direction. This assists an optimal positioning of the femur in the prosthetic socket. Hence, if the liner is a thigh liner the thickenings provide a soft tissue cushioning in the femur area and a better positioning of the femur in the prosthetic socket and reduce the pressure on the femur or thighbone. By providing a thickening on both the lateral and the medial side of the liner, the liner can be used for a left leg amputee as well as for a right leg amputee.

As already stated the at least one thickening can be formed by at least one pad arranged at the liner body. These pads can be prefabricated making it easier to produce a liner according to the present invention. The pad and the liner body can either be made from the same material, such as polyurethane or silicone or any other suitable polymer like material, or from different materials. In this case it is possible to use materials with different shore hardnesses.

In one embodiment the liner body is moulded to the at least one pad or the at least one pad is for example glued to the liner body.

In one embodiment of the invention the liner body comprises a knee portion having a pre-flexed angle. By this it is easily possible to determine which part of the liner body is intended to cover the head of the fibula and which part is intended to cover the shinbone. This is possible, because the position of the knee is predetermined to be in the knee portion. In addition, it is very easy to put on the liner in a reproducible manner such that it is ensured that the thickenings are arranged at their proper positions.

According to a preferred embodiment of the present invention, the liner body comprises at least two partial liners, in particular an inner liner and an outer liner. This has the advantage that different materials can also be used for the inner liner and the outer liner, the materials being adjusted according to the necessary properties in each case. In this way it is possible, for example, to use copolymer (TPE) as an inner liner, to which a skin-soothing white oil mixture has been added. In contrast to this, the outer liner can, for example, be made from a silicone that can be easily cleaned.

It has been shown to be especially advantageous if the at least one thickening is formed in one piece with one of the partial liners, or as a prefabricated pad. Furthermore, the idea of the invention of making the thickening and the liner body from different materials also covers an embodiment in which the liner body consists of two partial liners that are made from different materials, at least one of which comprising a one-piece thickening. A careful selection of the different materials makes it possible to separately choose the physical properties, such as elasticity, flexibility or hardness for the liner areas containing a thickening, and the remaining areas.

Should the at least one thickening be formed as a prefabricated pad, it can be attached to the inner liner or the outer liner. It is also possible to mould the pad to the liner material of the inner or outer liner, or to arrange the pad between the two partial liners, for example, and to connect it to at least one of the two partial liners using an adhesive or similar. All of these embodiments ensure that an additional cushioning is achieved at the desired points of the prosthetic liner. In addition, by means of the two different partial liners, the inner surface and the outer surface of the liner body can also be adapted to the individual needs and demands so they have the optimal effect.

A method for producing a liner according to the present invention comprises the step of forming at least one thickening in the first region and/or in the second region. This forming can comprise the steps of
a) positioning at least one prefabricated pad at a mould core,
b) applying a layer of liner body material,
c) hardening of the liner body material.

The step of applying the layer of liner body material may include immersing the mould core and the positioned pads into the liner body material. Alternatively the applying of the layer of liner body material includes injection moulding, casting or dipping of the liner body material.

In another embodiment of the method according to the present invention the forming the at least one thickening includes the step of applying at least one prefabricated pad onto an outer surface and/or onto an inner surface of the liner body. This can be done for example by gluing the pad on the surface of the liner body. This has the advantage that a standard liner body can be used onto which the pad is glued. This can be done in an individual form for each user of the respective liner. Alternatively the at least one thickening is integrally formed with the liner body, in particular by injection moulding.

Particularly during the production of the prosthetic liner, according to an embodiment of the present invention whose liner body comprises two partial liners, in particular an inner liner and an outer liner, various methods can be used. It has been shown to be advantageous if the method comprises the following steps:
- Producing a first partial liner that comprises the at least one thickening,
- Arranging a second partial liner on the first partial liner.

By splitting the method into these individual steps, an especially simple, quick and cost-effective implementation is achieved.

In a preferred embodiment the at least one thickening is produced in one piece with the first partial liner, by means of injection moulding, moulding or submersion. Alternatively, it may be arranged, in particular glued, on to the first partial liner in the form of a prefabricated pad. The production method selected each time depends on the desired properties of the respective partial liner with the attached thickening. For example, should the partial liner with this thickening be formed in one piece or made from the same material, the partial liner and thickening can be produced at the same time, for example by means of moulding or dipping in a shared mould. This has the advantage that only a small number of simple steps that are easily carried out are needed in the process. However, if the partial liner and the at least one thickening are made from different materials, the thickening can be initially produced in the form of a prefabricated pad. This is subsequently arranged on the first partial liner, for example by gluing it on. Alternatively it is also possible to arrange the prefabricated pad in a mould in such a way that the mould is subsequently filled with a partial liner material. In this way it is possible to mould the at least one prefabricated pad to the liner material. It is of course also possible to arrange the at least one prefabricated pad on a dip mould that is then submerged in the fluid material of the first partial liner.

If several thickenings should be arranged onto the first partial liner, it is also possible to create a single piece with at least one thickening and the partial liner and to form at least one further thickening as a prefabricated pad. This is then especially useful if the various thickenings should have different properties and thus must be made from different materials.

It has been shown to be advantageous if the second partial liner, in the form of at least one layer of partial liner material, is arranged in liquid form on the first partial liner, in particular by means of injection moulding, moulding or submersion, and the at least one layer of partial liner material subsequently hardening. For this purpose, the first partial liner may be arranged with the at least one thickening in a mould, which is then filled with the partial liner material of the second partial liner. Alternatively it is of course possible to submerge the first partial liner with the at least one thickening in a fluid material of the second partial liner. In all of these methods, it is an advantage that the material of the second partial liner, in the form of a fluid, is laid on the first partial liner with the at least one thickening, so that it results in an especially good and stable connection between the two partial liners.

Alternatively it is of course possible to fix the second partial liner to the first partial liner, in particular by gluing it on. The advantage of this process is that the two partial liners can be produced in parallel to one another, since they are not connected until they are fully complete.

In an alternative embodiment of the method, it is advantageous if it comprises the following steps:
- Producing a first partial liner
- Arranging a second partial liner onto the first partial liner, the second partial liner comprising the at least one thickening.

In contrast to the previously described processes, the first partial liner is initially produced without a thickening. This is made especially simple by means of a mould process or submersion process. Once the first partial liner has been produced in this way, the second partial liner is arranged onto it, the second partial liner now comprising the at least one thickening. It is also possible here, for example, that the first partial liner, which is already complete, is arranged in a mould that is then filled with the material of the second partial liner. As a result of the geometric arrangement and contour of this mould, the second partial liner and the at least one thickening can, for example, be manufactured in one piece. This is particularly useful if the at least one thickening and the second partial liner are made form the same material.

Alternatively or additionally to this, it is of course possible to arrange at least one prefabricated pad onto the first partial liner, for example, which will form the thickening. During the subsequent filling of the mould in which the first partial liner and the pads arranged upon it, is positioned, the pads are moulded to the material of the second partial liner, so that a particularly good connection occurs. With this production method, the at least one pad forms the thickening that is arranged between the two partial liners on the complete liner body. Alternatively or additionally to this, it is of course also possible to obtain the at least one thickening by means of a pad that is, for example, arranged on a mould in the proper position, in which the first partial liner is also positioned. In an especially simple embodiment, the first partial liner is the inner liner upon which the outer liner is fixed in a subsequently step. In this embodiment, the material of the outer liner is poured in between the inner liner and the pads which form the respective thickenings.

This can of course also happen in the form of injection moulding, moulding or submersion, or by another method, whereby the liner material of the second partial liner, in the form of a fluid, is put onto the first partial liner and subsequently hardens. The hardening can be achieved by means of simple drying, without the need for further steps in the method, or through steps influencing and accelerating the drying process. The actual embodiment of these steps in the method depends on the material used.

In an alternative, simple production method, a second prefabricated partial liner is arranged on a first prefabricated partial liner. This may occur by gluing it, for example. Here, the second partial liner in particular comprises the at least one thickening.

In another embodiment of the method, a first partial liner and a second partial liner are arranged on one another, with at least one prefabricated pad being positioned between them. With this method it is also possible to use three different materials: the inner liner and the outer liner may be made from different materials, while a third material is possible for the at least one pad that forms the thickening.

A connection between the two partial liners and/or the prefabricated pads can be made from different materials. It can be achieved by textiles of textile fibres arranged between the respective two parts or by a structure in one of the respective surfaces. This is for example described in US 2009/240344. It can also be achieved by a CVD-coating of at least one of the respective surfaces, by using a primer or bonding agent and/or by modifying the respective surface for example by a plasma treatment.

### Brief description of the drawings

Figure 1 is a schematic three-dimensional view of a below knee amputee prosthetic liner according to one embodiment of the present invention;
Figure 2 is a schematic three-dimensional view of the liner of figure 1 from another point of view;
Figure 3 is a schematic three-dimensional view of the liner of figure 1 and 2 from a third point of view;
Figure 4 is another schematic three-dimensional view of a liner according to a second embodiment of the present invention;
Fig. 5 is a schematic sectional view through a liner according to a further embodiment of the present invention;
Fig. 6 is a further sectional view through a liner according to a further embodiment of the present invention;
Fig. 7 is a sectional view of a liner according to a further embodiment of the present invention with pads on the outside of the liner body;
Fig. 8 is a sectional view of the liner according to a further embodiment of the present invention with pads on the inside of the liner body;
Fig. 9-12 are schematic sectional views through various liners according to various embodiments of the present invention;
Fig. 13 is a schematic sectional view through a thigh liner not covered by the present invention.

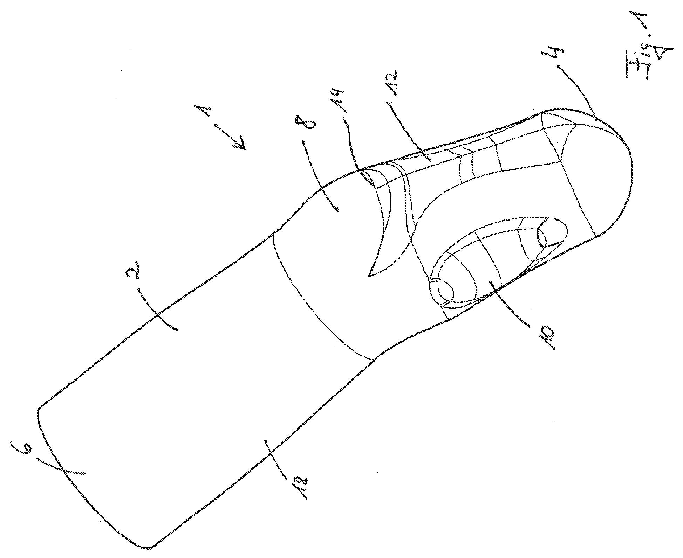

Fig. 1 shows a three-dimensional view of a liner 1 according to a first embodiment of the present invention. In the case shown the liner 1 is a liner 1 for a below knee prosthesis so that the amputation limb comprises a knee. Fig. 1 shows a liner body 2 having a closed distal end 4 and an open proximal end 6. The proximal end 6 is adapted to receive an amputation limb of a person. The liner body 2 comprises a knee part 8 having a pre-flexed angle.

The liner 1 according to the embodiment shown in Fig. 1 has a first thickening 10 in a first region of the liner body 2. The first region is intended to cover the head of the fibula of the person. The first thickening 10, which is in Fig 1 a prefabricated pad connected to the liner body 2 for example by one of the previously mentioned methods, cushions the head of the fibula when the amputation limb is received in the liner 1 and the liner 1 is worn by the amputee.

In addition the liner 1 shown in Fig. 1 has a second thickening 12, which is positioned in a second region of the liner 1. This second thickening 12 is intended to cover the shinbone of the amputation limb when the liner 1 is worn by the amputee. Determining the first and second region of the liner 1 as well as positioning the first thickening 10 and the second thickening 12 is easily possible, since the position of the knee is determined by the knee part 8 and in particular by the pre-flexed angle.

As can be seen in Fig. 1 the second thickening 12 has a curved upper edge 14 which is adapted to follow the shape of a patella of the amputation limb. This leads to an optimally positioned second thickening 12 and an optimal protection of the shinbone without affecting the patella.

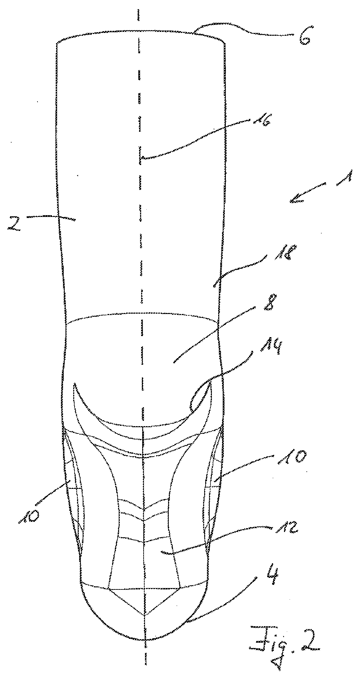

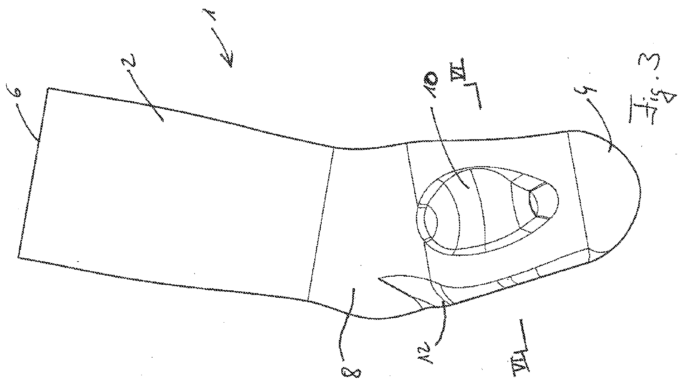

Fig. 2 shows the liner 1 of Fig. 1 from another point of view. From Fig. 2 is becomes clear that the liner 1 has two first thickenings 10, one of which is positioned on the right hand side of the second thickening 12 and the other one is positioned on the left hand side of the second thickening 12. It is therefore possible to use the liner 1 for both an amputation limb of a right leg and a left leg. Then dashed line in Fig. 2 indicates a plane of symmetry 16 with respect to which the liner 1 is symmetric. Fig. 3 shows a side view of the liner 1. The first thickenings 10 are positioned on the lateral and medial side of the liner 1.

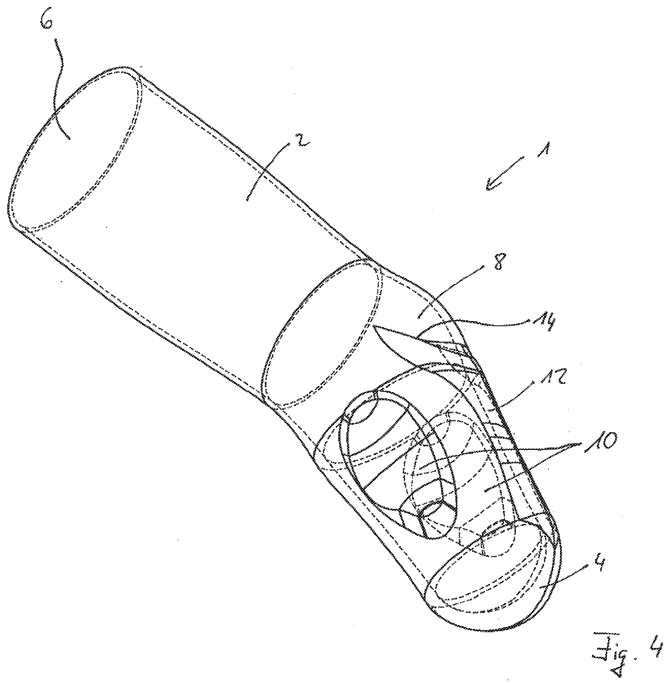

Fig 4. shows another three-dimensional view of the liner 1. The parts that are not visible from this point of view are shown in dashed lines. Again two first thickenings 10 are shown. Between these first thickenings 10 a second thickening 12 with the curved upper edge 14 is positioned. The thickness of the liner body 2 varies along the liner body 2. It is smallest at the open proximal end 6 and increases towards the closed distal end 4. By this the high pressures in this area when a person wears the liner 1 and walks with a prosthesis are cushioned to make the prosthesis more comfortable to wear.

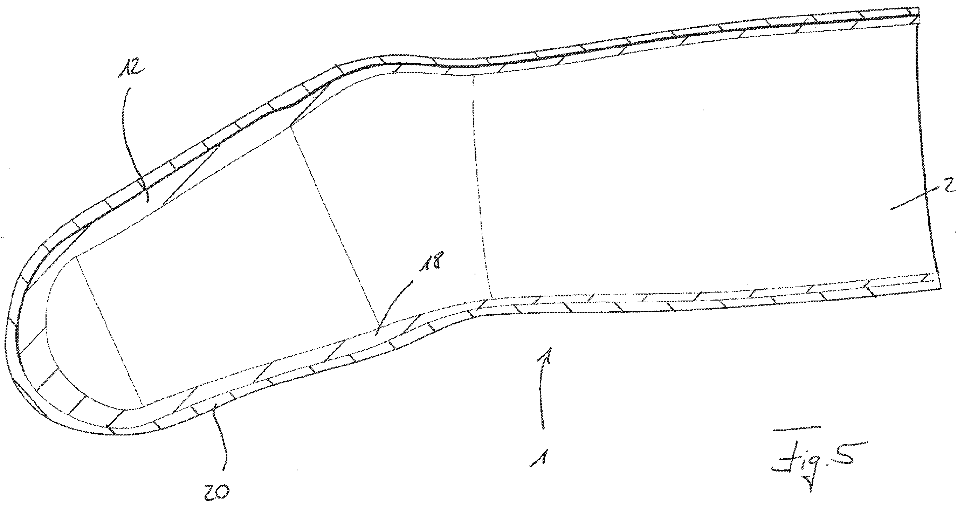

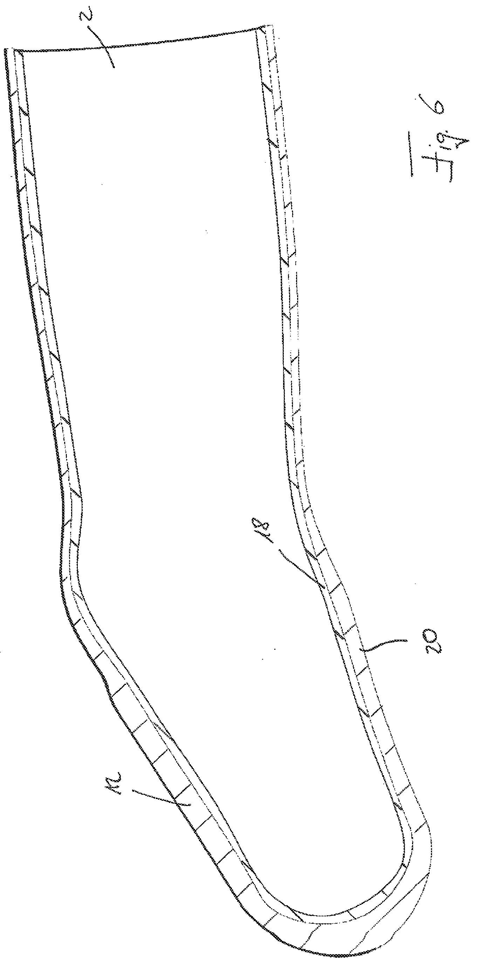

Figures 5 and 6 show sectional views through the liner 1, the liner body 2 now having an inner liner 18 and an outer liner 20. In Fig. 5 it is clear to see that the second thickening 12, which covers the wearer's shinbone, is designed to be the thickening of the inner liner 18. The outer liner 20 has almost the same thickness over the entire liner body 2.

Figure 6 shows a similar situation in which the liner body 2 is again made up of an inner liner 18 and an outer liner 20. However, in this case the inner liner 18 has almost the same thickness over the entire liner body 2, while the second thickening 12 should be seen as a thickening of the outer liner 20. It is of course possible to have combinations whereby the inner liner 18, as well as the outer liner 20, comprises thickenings.

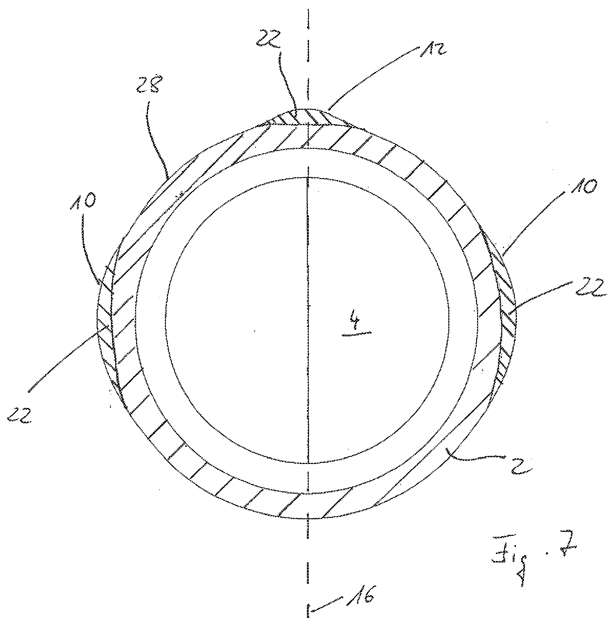

Fig. 7 shows a sectional view again along the line VI-VI in Fig. 3. The two first thickenings 10 and the second thickening 12 are not integrally formed with the liner body 2, but consist of three pads 22, which in the embodiment according to Fig. 7 are glued on the outer surface 26 of the liner body 2.

Alternatively the pads 22 may be arranged at an inner surface 28 of the liner body 2. To position the pads 22 at the inner surface 28 the pads 22 are arranged at a mould core in a first step. The mould core represents the amputation limb that is to be covered by the liner 1. After this, at least one layer of liner body material is applied to the mould core, for example by immersing the mould core and the pads 22 into a bath of this material. Alternatively the liner body material can be moulded or filled into a form, in which the mould core and the pads 22 are arranged.

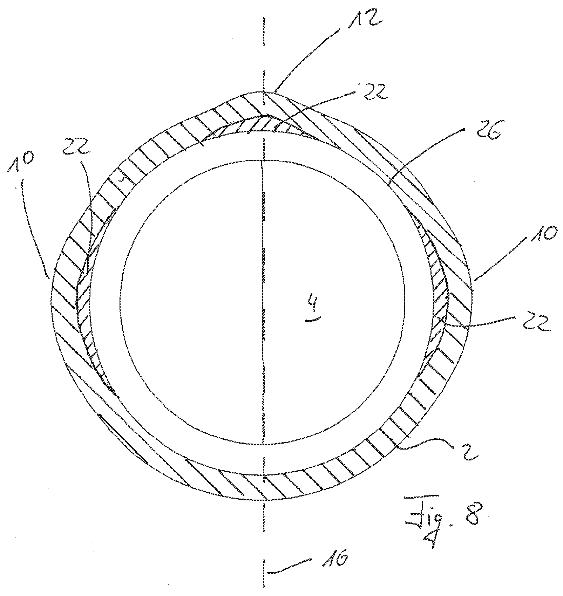

This situation is shown in Fig. 8, which again shows a sectional view along the line VI-VI in Fig. 3. The pads 22 are arranged at the inner surface 28 of the liner body 2.

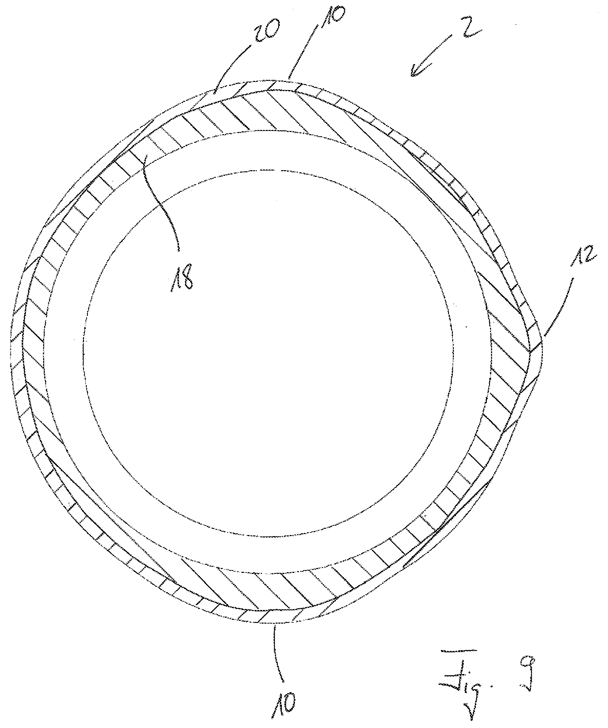

Fig. 9 to 12 show various cross-sections through a liner 1 along the line VI-VI in Fig. 3.

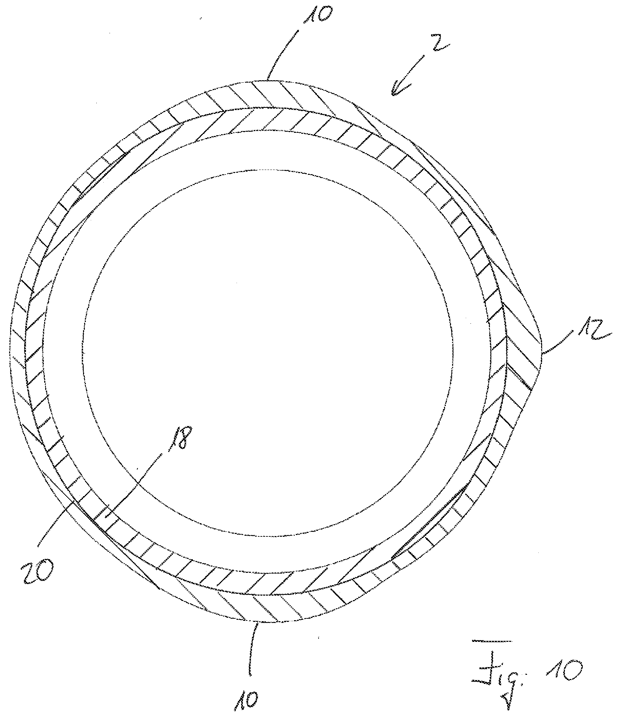

The inner liner 18 and the outer liner 20 can each be seen on the liner body 2 shown in Fig. 9 to 11. On the liner according to Fig. 9, the second thickening 12, as well as the two first thickenings 10, are created by thickenings of the inner liner 18. Even in the non-thickened parts between the first thickening 10 and the second thickening 12, the inner liner 18 is the stronger one of the partial liners 18, 20, i.e. it has a greater thickness. In contrast, in Fig. 10, the first thickenings 10 and the second thickening 12 are caused by thickenings in the outer liner 20.

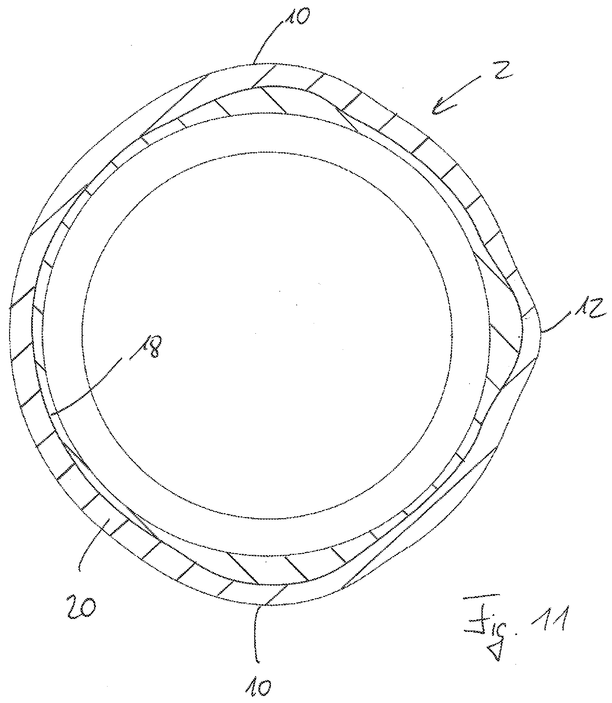

Fig. 11 again shows the situation in which the first thickenings 10 and the second thickening 12 are caused by thickenings of the inner liner 18, which now has a considerably smaller thickness in the non-thickened areas than the outer liner 20. As a result of these different embodiments, various requirements for the liner 1 can be met. In this way, the liner 1 can be adapted to the individual needs of the person wearing the prosthetic liner 1.

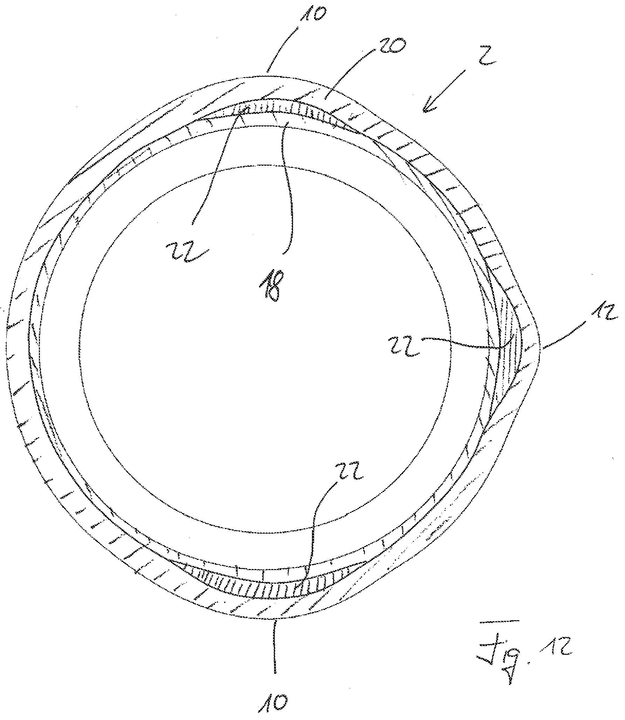

Fig. 12 shows a cross-section through a liner body 2 that comprise both an inner liner 18 and an outer liner 20. However, both partial liners 18, 20 have almost the same strength and thickness over the whole area of the liner body 2. The first thickenings 10 and the second thickening 12 are caused by pads 22 that have been arranged between the inner liner 18 and the outer liner 20 by means of one of the above described methods. In this way, it is possible to use three different materials for the liner body 2 and the thickenings, so that a further individualisation and adaptation to the particular needs is possible.

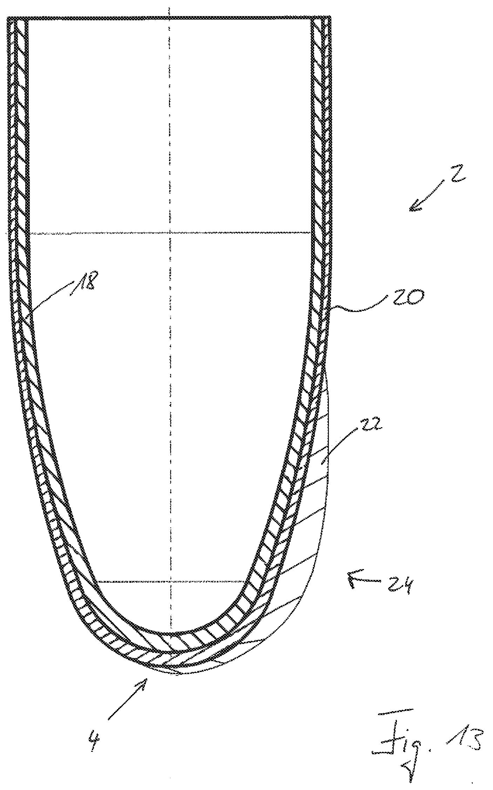

Fig. 13 shows a longitudinal section through a liner 2 that takes the form of a thigh liner in the embodiment depicted which is not covered by the present invention. The liner 2 comprises an inner liner 18 and an outer liner 20. It can be seen that the thickness of the inner liner 18 and the outer liner 20 increases towards the distal end 4 of the liner 2. A first cushioning is achieved as a result. In addition the liner 2 has a thickening 24, which is formed by a pad 22 that is designed as a separate component and which is integrally connected with at least one partial liner. The pad 22 can be arranged at the inner surface or between the two partial liners.

### List of references

- 1: liner
- 2: liner body
- 4: distal end
- 6: proximal end
- 8: knee part
- 10: first thickening
- 12: second thickening
- 14: upper edge
- 16: plane of symmetry
- 18: inner liner
- 20: outer liner
- 22: pad
- 24: thickening
- 26: outer surface
- 28: inner surface

## Claims

1. A below knee amputee prosthetic liner (1), comprising
a moulded polymer like liner body (2) for receiving an amputation limb of a person,
the liner body (2) having a closed distal end (4) and an open proximal end (6),
wherein the liner (1) comprises a first thickening (10) in a first region, and a second thickening (12) in a second region,
wherein the first region is adapted to cover a head of the fibula of the amputation limb and the first thickening (10) is provided on the lateral side of the liner body (2), such that the first thickening (10) is adapted to cover the head of the fibula once the amputation limb is received in the liner body (2), wherein the second region is adapted to cover the shinbone of the amputation limb, such that the second thickening (12) is adapted to cover the shinbone once the amputation limb is received in the liner body (2)
and wherein the liner body (2) and the thickenings (10, 12) are made from different materials, **characterized in that** the liner (1) is symmetric with respect to a sagittal plane and a further first thickening (10) is provided in the first region on the medial side of the liner body (2).

2. The liner of claim 1, wherein the at least one thickening is formed by at least one pad (22) arranged at the liner body.

3. The liner of claim 1 or 2 , wherein the liner body comprises a knee portion having a pre-flexed angle.

4. The liner of one of the preceding claims, wherein the liner body comprises at least two partial liners (18, 20), in particular an inner liner (18) and an outer liner (20).

5. The liner of claim 4, wherein the at least one thickening is formed in one piece with one of the partial liners, or as a prefabricated pad (22).

6. A method for producing the linear (1) of one of the preceding claims,
comprising the step of forming two first thickenings (10) in the first region and a second thickening (12) in the second region.

7. The method of claim 6, wherein the forming of the at least one thickening comprises:
a. Positioning at least one prefabricated pad at a mould core
b. Applying at least one layer of a liner body material
c. Hardening of the liner body material.

8. The method of claim 7, wherein the step of applying the layer of liner body material includes injection moulding, moulding, casting or dipping of the liner body material.

9. The method of one of the claims 6 to 8, wherein forming the at least one thickening includes applying, in particular gluing, at least one prefabricated pad onto an outer surface and/or an outer surface of the liner body.

10. The method of claim 6 comprising the following steps:
- Producing a first partial liner (18) that comprises the at least one thickening
- Arranging a second partial liner (20) on the first partial liner.

11. The method of claim 10, wherein the at least one thickening is formed in one piece with the first partial liner by injection moulding, moulding or submerging or the at least one thickening is arranged, in particular glued, to the first partial liner in form of a prefabricated pad (22).

12. The method of claim 10 or 11, wherein the second partial liner is arranged in liquid form on the first partial liner in form of at least one layer of partial liner material in particular by means of injection moulding, moulding or submersion wherein the partial liner material subsequently hardening.

13. The method of claim 10 or 11, wherein the second partial liner is fixed to the first partial liner, in particular by gluing it on.

14. The method of claim 6, comprising the following steps:
- Producing a first partial liner,
- arranging a second partial liner onto the first partial liner, the second partial liner comprising the at least one thickening.

15. The method of claim 14, wherein at least one prefabricated pad is arranged onto the first partial liner before the second partial liner is arranged on the first partial liner in form of at least one liquid layer of partial liner material in particular by means of injection moulding, moulding or submersion wherein the partial liner material subsequently hardening.

16. The method of claim 14, wherein a prefabricated second partial liner is arranged, in particular glued, to the first partial liner, the second partial liner comprising the at least one thickening.

17. The method of claim 6, wherein a first partial liner and a second partial liner are arranged on one another, with at least one prefabricated pad being positioned between them.

## Patentansprüche

1. Unterschenkelprothesenliner (1), umfassend
einen gegossenen polymerartigen Linerkörper (2) zum Aufnehmen eines Amputationsstumpfes einer Person,
wobei der Linerkörper (2) ein geschlossenes distales Ende (4) und ein offenes proximales Ende (6) aufweist,
wobei der Liner (1) eine erste Verdickung (10) in einer ersten Region und eine zweite Verdickung (12) in einer zweiten Region aufweist,
wobei die erste Region angepasst ist, um das Fibulaköpfchen des Amputationsstumpfes zu überdecken und die erste Verdickung an der lateralen Seite des Linerkörpers (2) vorgesehen ist, so dass die erste Verdickung (10) eingerichtet ist, das Fibulaköpfchen zu überdecken, sobald der Amputationsstumpf im Linerkörper (2) aufgenommen ist,
wobei die zweite Region angepasst ist, um das Schienbein des Amputationsstumpfes zu überdecken, so dass die zweite Verdickung (12) eingerichtet ist, das Schienbein zu überdecken, sobald der Amputationsstumpf im Linerkörper (2) aufgenommen ist,
und wobei der Linerkörper (2) und die Verdickungen (10, 12) aus verschiedenen Materialien hergestellt sind,
**dadurch gekennzeichnet, dass** der Liner (1) relativ zur Sagittalebene symmetrisch ausgebildet ist und eine weitere erste Verdickung (10) in der ersten Region auf der medialen Seite des Linerkörpers (2) vorgesehen ist.

2. Liner nach Anspruch 1, wobei die wenigstens eine Verdickung durch wenigstens ein Kissen (22) gebildet ist, das an dem Linerkörper angeordnet ist.

3. Liner nach Anspruch 1 oder 2, wobei der Linerkörper einen Knieanteil aufweist, der einen vorgebogenen Winkel aufweist.

4. Liner nach einem der vorstehenden Ansprüche, wobei der Linerkörper wenigstens zwei Teilliner (18, 20), insbesondere einen Innenliner (18) und einen Außenliner (20) aufweist.

5. Liner nach Anspruch 4, wobei die wenigstens eine Verdickung einstückig mit einem der Teilliner oder als vorgefertigtes Kissen (22) ausgebildet ist.

6. Verfahren zum Herstellen des Liners (1) nach einem der vorstehenden Ansprüche, bei dem zwei erste Verdickungen (10) in der ersten Region und eine zweite Verdickung (12) in der zweiten Region gebildet werden.

7. Verfahren nach Anspruch 6, wobei das Bilden der wenigstens einen Verdickung folgendes umfasst:
a. Positionieren wenigstens eines vorgefertigten Kissens in einem Formkern,
b. Aufbringen wenigstens einer Schicht aus einem Linerkörpermaterial,
c. Aushärten des Linerkörpermaterials.

8. Verfahren nach Anspruch 7, wobei das Aufbringen der Schicht aus Linerkörpermaterial Spritzgießen, Gießen, Formen oder Tauchen des Linerkörpermaterials beinhaltet.

9. Verfahren nach einem der Ansprüche 6 bis 8, wobei das Bilden der wenigstens einen Verdickung das Anbringen, insbesondere Kleben, wenigstens eines vorgefertigten Kissens an eine äußere Oberfläche und/oder eine innere Oberfläche des Linerkörpers beinhaltet.

10. Verfahren nach Anspruch 6, das die folgenden Schritte aufweist:
- Herstellen eines ersten Teilliners (18), der die wenigstens eine Verdickung beinhaltet,
- Anordnen eines zweiten Teilliners (20) am ersten Teilliner.

11. Verfahren nach Anspruch 10, wobei die wenigstens eine Verdickung einstückig mit dem ersten Teilliner durch Spritzgießen, Umformen oder Tauchen hergestellt wird oder die wenigstens eine Verdickung an dem ersten Teilliner in Form eines vorgefertigten Kissens (22) angeordnet, insbesondere angeklebt wird.

12. Verfahren nach Anspruch 10 oder 11, wobei der zweite Teilliner in flüssiger Form an dem ersten Teilliner in Form wenigstens einer Schicht des Teillinermaterials insbesondere durch Spritzgießen, Umformen oder Tauchen gebildet wird, wobei das Teillinermaterial anschließend aushärtet.

13. Verfahren nach Anspruch 10 oder 11, wobei der zweite Teilliner an dem ersten Teilliner befestigt wird, insbesondere, indem er angeklebt wird.

14. Verfahren nach Anspruch 6, das die folgenden Schritte aufweist:
- Herstellen eines ersten Teilliners,
- Anordnen eines zweiten Teilliners an dem ersten Teilliner, wobei der zweite Teilliner die wenigstens eine Verdickung beinhaltet.

15. Verfahren nach Anspruch 14, wobei wenigstens ein vorgefertigtes Kissen an dem ersten Teilliner angeordnet wird, bevor der zweite Teilliner am ersten Teilliner in Form wenigstens einer flüssigen Schicht eines Teillinermaterials insbesondere durch Spritzgießen, Umformen oder Tauchen angeordnet wird, wobei das Teillinermaterial anschließend aushärtet.

16. Verfahren nach Anspruch 14, wobei ein vorgefertigter zweiter Teilliner an dem ersten Teilliner angeordnet, insbesondere angeklebt wird, wobei der zweite Teilliner die wenigstens eine Verdickung aufweist.

17. Verfahren nach Anspruch 6, wobei der erste Teilliner und der zweite Teilliner aneinander angeordnet werden, wobei wenigstens ein vorgefertigtes Kissen zwischen beiden positioniert wird.

## Revendications

1. Doublage de prothèse (1) pour une personne amputée au-dessous du genou, comprenant
un corps de doublage en polymère moulé (2) pour recevoir un membre amputé d'une personne,
le corps de doublage (2) ayant une extrémité distale fermée (4) et une extrémité proximale ouverte (6),
dans lequel le doublage (1) comprend un premier épaississement (10) dans une première région et un second épaississement (12) dans une seconde région,
dans lesquelles la première région est adaptée à couvrir une tête de la fibule du membre amputé et le premier épaississement (10) est prévu sur le côté latéral du corps de doublage (2), de sorte que le premier épaississement (10) est adapté à couvrir la tête de la fibule une fois que le membre amputé est reçu dans le corps de doublage (2),
dans lesquelles la seconde région est adaptée à couvrir le tibia du membre amputé, de telle façon que le second épaississement (12) est adapté à couvrir le tibia une fois que le membre amputé est reçu dans le corps de doublage (2),
et dans lequel le corps de doublage (2) et l'épaississement (10, 12) sont réalisés de matériaux différents,
**caractérisé en ce que**
le doublage (1) est symétrique par rapport à un plan sagittal et un autre premier épaississement (10) est prévu dans la première région sur le côté médial du corps de doublage (2).

2. Doublage selon la revendication 1, dans lequel ledit au moins un épaississement est formé par au moins un coussin (22) agencé sur le corps de doublage.

3. Doublage selon la revendication 1 ou 2, dans lequel le corps de doublage comprend une portion de genou ayant un angle de préflexion.

4. Doublage selon l'une des revendications précédentes, dans lequel le corps de doublage comprend au moins deux doublages partiels (18, 20), en particulier un doublage intérieur (18) et un doublage extérieur (20).

5. Doublage selon la revendication 4, dans lequel ledit au moins un épaississement est formé d'une seule pièce avec l'un des doublages partiels, ou sous forme de coussin préfabriqué (22).

6. Procédé pour produire le doublage (1) de l'une des revendications précédentes,
comprenant l'étape consistant à former deux premiers épaississements (10) dans la première région et un second épaississement (12) dans la seconde région.

7. Procédé selon la revendication 6, dans lequel la mise en forme dudit au moins un épaississement comprend :
a) le positionnement d'au moins un coussin préfabriqué sur un noyau de moule
b) l'application d'au moins une couche d'un matériau de corps de doublage
c) le durcissement du matériau de corps de doublage.

8. Procédé selon la revendication 7, dans lequel l'étape consistant à appliquer la couche de matériau de corps de doublage inclut un moulage par injection, un moulage, une coulée ou une immersion du matériau de corps de doublage.

9. Procédé selon l'une des revendications 6 à 8, dans lequel l'étape de formation dudit au moins un épaississement inclut d'appliquer, en particulier de coller, au moins un coussin préfabriqué sur une surface extérieure et/ou une surface extérieure du corps de doublage.

10. Procédé selon la revendication 6, comprenant les étapes suivantes :
- on produit un premier doublage partiel (18) qui comprend ledit au moins un épaississement, et
- on arrange un second doublage partiel (20) sur le premier doublage partiel.

11. Procédé selon la revendication 10, dans lequel ledit au moins un épaississement est formé d'une seule pièce avec le premier doublage partiel par moulage par injection, moulage ou immersion, ou bien ledit au moins un épaississement est agencé, en particulier collé, sur le premier doublage partiel sous la forme d'un coussin préfabriqué (22).

12. Procédé selon la revendication 10 ou 11, dans lequel le second doublage partiel est agencé sous forme liquide sur le premier doublage partiel, sous la forme d'au moins une couche de matériau de doublage partiel, en particulier au moyen d'un moulage par injection, d'un moulage ou d'une immersion, et dans lequel le matériau de doublage partiel est ensuite amené à durcir.

13. Procédé selon la revendication 10 ou 11, dans lequel le second doublage partiel est fixé sur le premier doublage partiel, en particulier en le collant sur celui-ci.

14. Procédé selon la revendication 6, comprenant les étapes suivantes :
- on produit un premier doublage partiel,
- on agence un second doublage partiel sur le premier doublage partiel, le second doublage partiel comprenant au moins un épaississement.

15. Procédé selon la revendication 14, dans lequel au moins un coussin préfabriqué est agencé sur le premier doublage partiel avant que le second doublage partiel soit agencé sur le premier doublage partiel sous la forme d'au moins une couche liquide de matériau de doublage partiel, en particulier au moyen d'un moulage par injection, d'un moulage ou d'une immersion, et dans lequel le matériau de doublage partiel est ensuite amené à durcir.

16. Procédé selon la revendication 14, dans lequel un second doublage partiel préfabriqué est agencé, en particulier collé, sur le premier doublage partiel, le second doublage partiel comprenant ledit au moins un épaississement.

17. Procédé selon la revendication 6, dans lequel un premier doublage partiel et un second doublage partiel sont agencés l'un sur l'autre, et dans lequel au moins un coussin préfabriqué est positionné entre eux.
